# EUROPÄISCHE PATENTANMELDUNG

(11) **EP 2 233 074 A1**
(43) Veröffentlichungstag der Anmeldung: **29.09.2010**
(21) Anmeldenummer: 10156174.4
(22) Anmeldetag: 11.03.2010
(51) Int. Cl.: A61B 5/107, G01B 11/24

(54) **Vorrichtung und Verfahren zum Messen der Distanz zum Trommelfell**

(30) Priorität: 23.03.2009 DE 102009014463
(71) Anmelder: Siemens Medical Instruments Pte. Ltd., Singapore 139959 (SG)
(72) Erfinder: Grafenberg, Alexander, Dr., 91090 Effeltrich (DE); Kunz, Martin, 81377 München (DE); Rass, Uwe, 90480 Nürnberg (DE); Wagner, Frank, 278636 Singapore (SG)
(74) Vertreter: Maier, Daniel Oliver

(57) **Zusammenfassung**

Die Erfindung betrifft eine Vorrichtung und ein Verfahren zum Messen der Distanz zum Trommelfell. Die Vorrichtung umfasst einen optischen Sensor (22) und eine Signalverarbeitungseinrichtung (23). Gemäß einem Grundgedanken der Erfindung erfasst der optische Sensor (22) ein Abbild des Trommelfells (12), und die Signalverarbeitungseinrichtung (23) ermittelt in dem Abbild eine quer zu der zu messenden Distanz (D) erstreckte Fläche (A1,A2) oder Abstand (d1,d2,d3) und ermittelt in Abhängigkeit von der ermittelten Fläche (A1,A2) oder Abstand (d1,d2,d3) die Distanz (D) zu Trommelfell (12). Das Verfahren umfasst gemäß einem Grundgedanken der Erfindung die Schritte (S1) Erfassen eines Abbilds des Trommelfells (12), (S2) in dem Abbild Ermitteln einer quer zu der zu messenden Distanz (D) erstreckten Fläche (A1,A2) oder Abstand (d1,d2,d3), und (S3) Ermitteln der Distanz (D) in Abhängigkeit von der ermittelten Fläche (A1,A2) oder Abstand (d1,d2,d3). In einer vorteilhaften Weiterbildung wird eine Projektionsabbildung auf oder in die Nähe des Trommelfells (12) projiziert; dann kann die Distanz (D) vorteilhaft aus dem Abstand (d1,d2,d3) zwischen der Projektionsabbildung (31) und dem Trommelfell (12) oder der Bildmitte des Abbilds ermittelt werden. Vorteilhafterweise kann ein eventuell zu anderen Zwecken ohnehin vorhandener optischer Sensor verwendet werden. Vorteilhafterweise stehen Bildverarbeitungsalgorithmen zur Analyse von Bilddaten hinsichtlich gewöhnlicher geometrischer Abbildungsmerkmale, nämlich Fläche oder Abstand, zur Verfügung und erfordern keine besondere Implementierung auf Vorrichtungsseite. Zudem arbeiten solche Bildverarbeitungsalgorithmen weitgehend unabhängig von Störungen der Bildqualität.

## Beschreibung

Die Erfindung betrifft eine Vorrichtung und ein Verfahren zum Messen der Distanz eines Instruments, das in einen Gehörgang eingeführt ist, zu dem Trommelfell des Gehörgangs. Instrumente werden in den Gehörgang zu verschiedensten Zwecken der Untersuchung und Behandlung eingeführt, häufig zur Untersuchung des Gehörapparats oder zur Behandlung von Hörschädigungen.

Beispielsweise werden Untersuchungen des äußeren Gehörgangs sowie des Trommelfells im Rahmen der Otoskopie mit Hilfe eines Otoskops vorgenommen. Ein Otoskop weist einen länglichen Tubus oder Schlauch zum Einführen in den äußeren Gehörgang auf, mittels dem entweder direkt oder durch Video-Übertragung Bilder aus dem Inneren des Gehörgangs gewonnen werden können. Das Einführen des Otoskops in den Gehörgang ist insbesondere deswegen erforderlich, weil der Gehörgang häufig verwinkelt oder gewunden ist, so dass eine direkte und geradlinige Betrachtung von außerhalb nicht möglich ist.

Im Rahmen der Hörhilfe-Therapie werden verschiedene Hörhilfe-Bauformen eingesetzt, die mit unterschiedlichen Vorrichtungskomponenten unterschiedlich weit in den Gehörgang eingeführt werden. Insbesondere sogenannte HdO-(Hinter-dem-Ohr)-Geräte weisen einen weit in den Gehörgang hineinreichenden Schlauch auf. Der Schlauch dient entweder dazu, akustische Signale von dem hinter dem Ohr des Hörhilfe-Trägers angeordneten Hörhilfe-Gehäuse in den Gehörgang zu leiten, oder stattdessen elektrische Signale zu einem an dem Schlauch im Gehörgang angeordneten Receiver zu führen. Der Schlauch eines HdO-Geräts kann unterschiedlich tief im Gehörgang platziert werden, wobei eine Patienten-individuelle Platzierung durch Versuche zu ermitteln sein kann.

Bei der Durchführung einer Otoskopie-Untersuchung, Platzierung eines HdO-Hörhilfe-Schlauchs oder bei sonstigen Manipulationen im Gehörgang ist es wichtig, nicht das Trommelfell des Patienten oder der betroffenen Person zu berühren. Berührungen des Trommelfells werden als unangenehm oder schmerzhaft empfunden. Zu diesem Zweck ist es erforderlich, den Abstand zwischen dem in den Gehörgang eingeführten Instrument und dem Trommelfell zu überwachen.

Die einfachste und übliche Art, diesen Abstand zu überwachen, ist eine optische Überwachung durch den Operateur oder Bediener des Instruments. Zum Beispiel kann bei einer Otoskopie-Untersuchung auf den durch das Otoskop gewonnenen Bildern das Trommelfell immer im Auge behalten werden. Bei der Platzierung von Hörhilfe-Komponenten, z.B. Receivern, oder Mess-Sonden kann ebenfalls eine optische Überwachung durch den Akustiker oder Operateur gemacht werden. Die optische Überwachung ist allerdings insoweit fehleranfällig, als zum einen die Überwachung des Abstands besondere Aufmerksamkeit des Operateurs erfordert und dieser abgelenkt sein kann. Zum anderen ist aufgrund der verzerrten optischen Darstellungs-Verhältnisse, sowohl was Beleuchtung als auch was Blickwinkel und Apertur betrifft, der Abstand des Instruments zum Trommelfell optisch häufig nur schwer einzuschätzen und insbesondere kurze Abstände sind in der Regel nur mit großer Erfahrung und Aufmerksamkeit erfassbar.

Um den Problemen der optischen Abstandsüberwachung durch den Operateur zu begegnen, ist aus der Druckschrift WO 02/16867 A1 ein Instrument bekannt, das einen Kollisions-Detektor aufweist. Das Instrument dient der optischen 3D-Erfassung des Gehörgangs und kann zu diesem Zweck in den Gehörgang eingeführt werden. Der Kollisions-Detektor ist am distalen Ende des Instruments angeordnet, das zudem aus weichem Kunststoffmaterial, z.B. Silikon, ausgeführt sein kann. Die Funktionsweise des Kollisions-Detektors ist nicht detailliert beschrieben.

Aus der Druckschrift DE 10 2006 057 099 A1 ist eine Holographie-Einheit zur Untersuchung des Gehörgangs vorbekannt. Die Holographieeinheit zeichnet optische Abbilder des Gehörgang-Inneren auf, aus denen ein 3D-Abbild des Gehörgangs gewonnen werden kann. Sobald das 3D-Abbild vorliegt, ist auch die Position der Holographieeinheit und somit deren Abstand zum Trommelfell bekannt. Eine automatische Abstandsüberwachung erfolgt jedoch nicht, so dass diese der Aufmerksamkeit des Operateurs unterliegt.

Aus der Druckschrift US 5,044,373 ist ein Instrument für akustische Messungen im Gehörgang bekannt. Das Instrument wird im Rahmen der Anpassung eines Hörgeräts an die individuellen Gegebenheiten beim jeweiligen Hörgeräte-Träger verwendet. Es umfasst eine Messprobe, die in den Gehörgang eingeführt werden kann. Die Messprobe weist einen Sensor zur Messung der Distanz von der Messprobe zum Trommelfell auf. Der Sensor kann unter Verwendung von Schallwellen, elektromagnetischen Wellen oder Licht arbeiten. Bei Verwendung von Licht wird entweder eine Laufzeitmessung am reflektierten Licht oder eine Messung der Signalstärke des reflektierten Lichts vorgenommen. Eine Laufzeitmessung kann hohe Messgenauigkeit gewährleisten; sie ist jedoch verhältnismäßig aufwendig in der Implementierung. Die Messung der Signalstärke ist mit relativ geringem Implementierungsaufwand implementierbar; die Messgenauigkeit ist jedoch verhältnismäßig niedrig und die Messmethode ist anfällig vor Verschmutzungen des Sensors.

Der Erfindung liegt die Aufgabe zugrunde, eine Messung eines Abstands zwischen einem in einen Gehörgang eingeführten Instrument und dem Trommelfell des Gehörgangs zu ermöglichen, die automatisiert abläuft und eine ausreichende Messgenauigkeit gewährleistet, um vor Berührungen des Trommelfells verlässlich warnen zu können. Zudem soll die Methode mit geringem Aufwand und angesichts des geringen, im Gehör verfügbaren Raums raumsparend implementierbar sein.

Gemäß einem Grundgedanken der Erfindung umfasst eine Vorrichtung zur Messung einer Distanz zwischen dem distalen Ende eines Instruments, das zum Einführen in einen Gehörgang ausgebildet ist, und einem endständigen Abschluss, insbesondere dem Trommelfell, des Gehörgangs, in den das Instrument eingeführt ist, einen optischen Sensor und eine Signalverarbeitungseinrichtung. Gemäß diesem Grundgedanken der Erfindung erfasst der optische Sensor ein Abbild des endständigen Abschlusses. Die Signalverarbeitungseinrichtung ermittelt in dem Abbild eine quer zu der zu messenden Distanz erstreckte Fläche oder Abstand und ermittelt in Abhängigkeit von der ermittelten Fläche oder Abstand die Distanz. Mit quer ist dabei zum Ausdruck gebracht, dass der optische Sensor im wesentlichen in Richtung der zu messenden Distanz und die Bildebende des Abbilds daher nicht parallel zu der zu messenden Distanz ausgerichtet ist.

Ein wesentliches Merkmal dieses Grundgedankens besteht darin, dass ein ohnehin vorhandener optischer Sensor verwendet werden kann, da die Sensorbilddaten hinsichtlich gewöhnlicher geometrischer Abbildungsmerkmale, nämlich Fläche oder Abstand, analysiert werden. Damit können Abbilder im sichtbaren Wellenlängenbereich und ein in diesem Bereich arbeitender Sensor verwendet werden, der zum Beispiel an einem Otoskops oder sonstigen Instrument zu anderen Zwecken ohnehin vorhanden sein kann. Optische Sensoren, z.B. Miniatur-Bildsensoren oder Sensoraufbauten unter Verwendung von Glasfaserkabeln, die für eine Anwendung im Gehörgang ausreichend klein sind, sind ohne weiteres verfügbar.

Zudem ist lediglich ein den Abstand ermittelnder Algorithmus in der Signalverarbeitungseinrichtung erforderlich und weitere Anpassungen sind nicht erforderlich. Geeignete Bildverarbeitungsalgorithmen sind ohne weiteres verfügbar. Besonders vorteilhaft ist die geringe Fehleranfälligkeit bei der Ermittlung und Analyse geometrischer Bilddaten, das sie nur wenigen Störeinflüssen unterliegen. Gerade bei Verwendung eines ohnehin zu anderen Zwecken vorgesehenen optischen Sensors wird die Benutzungssicherheit zusätzlich erhöht, weil massive Bildstörungen, die den Bildverarbeitungsalgorithmus stören könnten, auch vom Operateur wahrgenommen würden, der somit über eventuelle Probleme aufgrund von Bildstörungen bei der Bildverarbeitung laufend informiert ist.

Nicht zuletzt ist auch die vorgeschlagene Analyse der Abstandsinformation aus den Bilddaten auch unaufwendig implementierbar, da keine zusätzlichen Vorrichtungsbestandteile vorgesehen werden müssen, und da Abstände und Flächen in der Bilddatenverarbeitung allein durch einen geeigneten Bilddatenverarbeitungsalgorithmus ermittelbar sind. Begünstigend kommt hinzu, dass die zu analysierenden Bilder hauptsächlich auf bekannten und für sämtliche Patienten ähnlichen Grundstrukturen und Farben beruhen, deren geringe Variabilität die Bilddatenanalyse wesentlich vereinfacht.

Gemäß einer vorteilhaften Weiterbildung des Grundgedankens ist die optische Apertur des Sensors konstant. Die Bedeutung der Apertur des optischen Sensors besteht darin, dass jegliche Änderung der Apertur auch eine Änderung des Abbildungsmaßstabes bewirkt. Um bei der Bilddatenanalyse zur Ermittlung der Trommelfell-Distanz von gleichbleibenden Voraussetzungen ausgehen zu können, ist daher eine konstante Apertur vorteilhaft, weil sie die Verarbeitung im Bilddatenverarbeitungsalgorithmus vereinfacht.

Gemäß einer weiteren vorteilhaften Weiterbildung ermittelt die Signalverarbeitungseinrichtung die Distanz in zusätzlicher Abhängigkeit von der optischen Apertur des Sensors. Sollen Bilddaten eines optischen Sensors verwendet werden, die mit variierenden Aperturen aufgenommen werden, z.B. weil der Operateur für den jeweiligen Untersuchungszweck Veränderungen der Apertur einstellt, verändern sich mit dem Abbildungsmaßstab selbstverständlich auch Abstände und Flächen in den gewonnenen Bilddaten, und zwar abhängig von Apertur-Änderungen. Derartige Veränderungen können in vorteilhafter Weise durch die Signalverarbeitungseinrichtung berücksichtigt werden, so dass eine zuverlässige Distanz-Analyse trotz Apertur-Änderungen möglich ist.

Gemäß einer weiteren vorteilhaften Weiterbildung ermittelt die Signalverarbeitungseinrichtung in dem Abbild eine Fläche des endständigen Abschlusses und ermittelt in Abhängigkeit von dieser Fläche die Distanz. Die Auswertung von Flächen in den optischen Bilddaten bietet sich insofern an, als das Trommelfell als verhältnismäßig einheitlich gefärbte und gestaltete Fläche gut erkennbar und vom umliegenden Gehörgang meist auch gut unterscheidbar ist. Damit kann eine BilddatenAnalyse eine zuverlässige Erkennung des Trommelfells und Unterscheidung vom umliegenden Gehörgang gewährleisten. Aus der Geometrie optischer Projektionen ist geläufig, dass - bei unveränderter Apertur bzw. Abbildungsmaßstab - aus geringerem Abstand größer erscheinen. Geht man von einer gleichbleibenden gesamten Abbildungsgröße aus, wird damit ein immer größerer Flächenanteil der Abbildung von dem bei abnehmender Distanz größer erscheinenden Trommelfeld eingenommen. Damit ist die Fläche der Bilddaten, die durch das Trommelfell eingenommen wird, umgekehrt ein Maß für die Distanz des optischen Sensors zum Trommelfell.

Gemäß einer weiteren vorteilhaften Weiterbildung ermittelt die Signalverarbeitungseinrichtung in dem Abbild eine den endständigen Abschluss nicht beinhaltende Fläche und ermittelt in Abhängigkeit von dieser Fläche die Distanz.

Gemäß einer weiteren vorteilhaften Weiterbildung umfasst die Vorrichtung einen optischen Projektor, der dazu ausgebildet ist, eine vorbestimmte Projektionsabbildung zu projizieren, und der so angeordnet ist, dass er die Projektionsabbildung in Richtung des endständigen Abschlusses des Gehörgangs projiziert, und die Signalverarbeitungseinrichtung ermittelt in dem Abbild einen Abstand zwischen der Projektionsabbildung und dem endständigen Abschluss und ermittelt in Abhängigkeit von diesem Abstand die Distanz zum Abschluss bzw. zum Trommelfell.

Die Verwendung einer vorbestimmten und gleichbleibenden Projektionsabbildung ermöglicht es in vorteilhafter Weise, ein absolutes Maß für die Distanz vom Sensor zum Trommelfell bzw. zur Projektionsfläche zu gewinnen. Denn selbst wenn bei Verwendung der Fläche des Trommelfells in der Abbildung ein relatives Maß der Distanz gegeben ist, könnte die absolute Distanz doch nur bei Kenntnis der tatsächlichen Fläche des Trommelfells und der Apertur ermittelt werden. Bei geeigneter Wahl der Projektionswinkel steht eine zusätzliche Information zur Verfügung, z.B. die bekannte Form und Größe der Projektionsabbildung und der bekannte Projektionswinkel, mit deren Hilfe die absolute Distanz ermittelt werden kann. Es kann, ggf. nach vorheriger Kalibrierung, einem bestimmten typischen Maß der Projektionsabbildung, z.B. einem Durchmesser oder einem Abstandsmaß, eine Distanz zugeordnet werden. Bei Projektions-Geometrien, die zur Ermittlung eines absoluten Distanz-Maßes nicht geeignet sind, trägt die Verwendung der Projektionsabbildung zur Erhöhung der Genauigkeit der Distanz-Ermittlung beitragen.

Wird die Projektionsabbildung nicht in Richtung der optischen Achse des Sensors projiziert, sondern in einem vorbestimmten und bekannten Winkel dazu (Parallaxe), so bildet der Versatz bzw. der Abstand der Projektionsabbildung zur Mitte des Abbilds ein Maß für die absolute Distanz.

Gemäß einer weiteren vorteilhaften Weiterbildung ist die vorbestimmte Projektionsabbildung in Anpassung an die erwartete Kontur des endständigen Abschlusses konturiert. Durch die Anpassung der Kontur der Projektionsabbildung kann diese besser auf die Kontur des Trommelfells ausgerichtet werden, was Fehler durch Fehlausrichtungen zu vermeiden hilft. Zudem erleichtern ähnliche Konturen das Erkennen und Analysieren typischer Abstandsmaße zwischen der Projektionsabbildung und dem Abschluss.

Gemäß einer weiteren vorteilhaften Weiterbildung ermittelt die Signalverarbeitungseinrichtung in dem Abbild einen Abstand zwischen einer Kontur der Projektionsabildung und einer Kontur des endständigen Abschlusses und ermittelt in Abhängigkeit von diesem Abstand die Distanz.

Gemäß einem weiteren Grundgedanken der Erfindung umfasst ein Verfahren zur Messung einer Distanz zwischen dem distalen Ende eines Instruments, das zum Einführen in einen Gehörgang ausgebildet ist, und einem endständigen Abschluss, insbesondere dem Trommelfell, eines Gehörgangs, in den das Instrument eingeführt ist, die Schritte (S1) Erfassen eines Abbilds des endständigen Abschlusses (S2) in dem Abbild Ermitteln einer quer zu der zu messenden Distanz erstreckten Fläche oder Abstand (S3) Ermitteln der Distanz in Abhängigkeit von der ermittelten Fläche oder Abstand.

Gemäß einer vorteilhaften Weiterbildung wird beim Ermitteln der Distanz zusätzlich eine Abhängigkeit von der optischen Apertur des zum Erfassen des Abbildes verwendeten optischen Sensors berücksichtigt.

Gemäß einer vorteilhaften Weiterbildung umfasst das Verfahren die Schritte (S4) in dem Abbild Ermitteln einer Fläche des endständigen Abschlusses (S5) Ermitteln der Distanz in Abhängigkeit von dieser Fläche.

Gemäß einer vorteilhaften Weiterbildung umfasst das Verfahren die Schritte (S6) in dem Abbild Ermitteln einer den endständigen Abschluss nicht beinhaltenden Fläche (S7) Ermitteln der Distanz in Abhängigkeit von dieser Fläche.

Gemäß einer vorteilhaften Weiterbildung umfasst das Verfahren die Schritte
(S8) Projizieren einer vorbestimmten Projektionsabbildung in Richtung des endständigen Abschlusses
(S9) in dem Abbild Ermitteln eines Abstands zwischen der Projektionsabbildung und dem endständigen Abschluss (S10) Ermitteln der Distanz in Abhängigkeit von diesem Abstand.

Gemäß einer vorteilhaften Weiterbildung ist die vorbestimmte Projektionsabbildung in Anpassung an die erwartete Kontur des endständigen Abschlusses konturiert.

Gemäß einer vorteilhaften Weiterbildung umfasst das Verfahren die Schritte (S11) in dem Abbild Ermitteln eines Abstands zwischen einer Kontur der Projektionsabildung und einer Kontur des endständigen Abschlusses (S12) Ermittelt der Distanz in Abhängigkeit von diesem Abstand.

Weitere vorteilhafte Weiterbildungen gehen aus den Patentansprüchen sowie der nachfolgenden Beschreibung von Ausführungsbeispielen anhand von Figuren hervor. Es zeigen:
- Fig. 1: ein Ohr mit Instrument,
- Fig. 2: ein Instrument mit Abbildungsprojektion,
- Fig. 3: einen Wahrnehmungswinkel bei abnehmendem Abstand,
- Fig. 4: eine Projektionsabbildung und ein Trommelfell und
- Fig. 5: Flächenverhältnisse Trommelfell ??

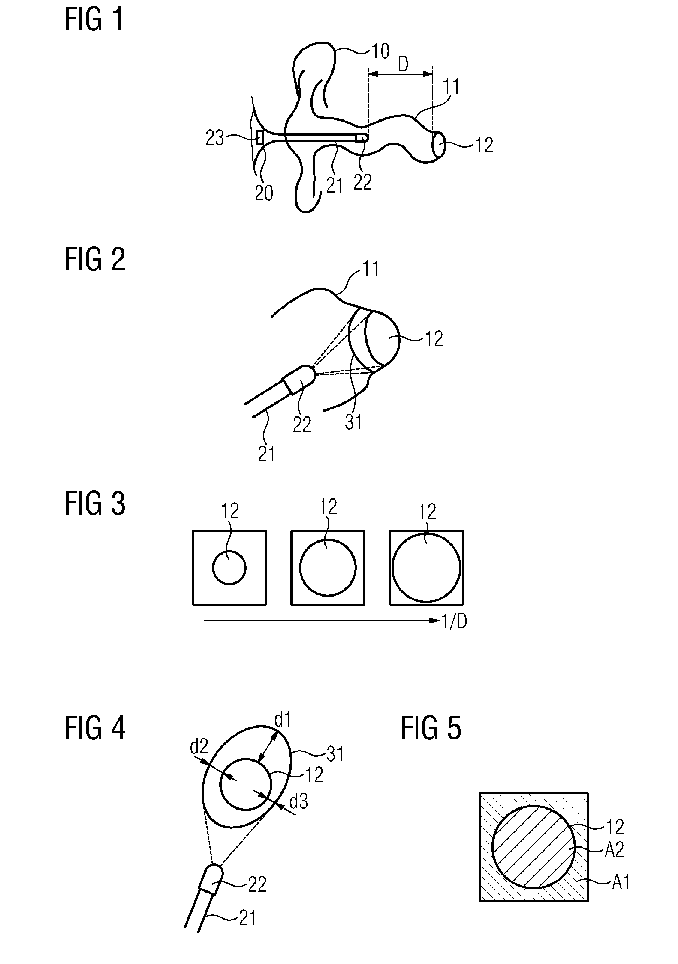

In **Fig. 1** ist ein Instrument 20 dargestellt, das in einen Gehörgang 11 teilweise eingeführt ist. Bei dem Instrument 20 kann es sich um ein Otoskop, eine akustische Messsonde oder jegliches sonstiges Untersuchungs- und Behandlungsinstrument handeln. Das Instrument 20 weist einen länglichen Tubus oder Schlauch 21 auf, der je nachdem fest oder flexibel ausgeführt sein kann. An dem distalen, d.h. vom Operateur oder Benutzer abgewandten, Ende des Instruments 20 ist ein optischer Sensor 22 angeordnet. Dies kann insbesondere im Falle eines Otoskops mit elektronischer Bilddaten-Gewinnung und Übertragung der Bildsensor des Otoskops sein. Es kann sich jedoch auch um einen eigens vorgesehenen Sensor handeln. Mit dem optischen Sensor in nicht dargestellter Weise verbunden ist eine Signalverarbeitungseinrichtung 23.

Der Gehörgang 11 ist gewinkelt oder gewunden und umfasst als endständigen Abschluss das Trommelfell 12. Das Trommelfell 12 trennt den Gehörgang 11 und damit das äußere Ohr vom nicht dargestellten Mittelohr und Innenohr. Berührungen des Trommelfells 12, beispielsweise durch das distale Ende des Instruments 20, werden vom Patienten als unangenehm oder gar schmerzhaft empfunden. Zudem besteht die Gefahr einer Beschädigung des Trommelfells 12.

Um Beeinträchtigungen oder Beschädigungen zu vermeiden, muss jederzeit eine ausreichende Distanz des distalen Endes des Instruments 20 zum Trommelfell 12 eingehalten werden. Der optische Sensor 22 wird daher in ein System zur automatischen Ermittlung der Distanz eingebunden. Das vom Sensor 22 erzeugte Abbild des im Gehörgang endständigen Trommelfells 12 wird durch die Signalverarbeitungseinrichtung 23 analysiert. Dabei werden Abstände oder Flächen innerhalb der Abbildung im Sinne der unten folgenden Erläuterungen ermittelt.

In **Fig. 2** ist der Tubus oder Schlauch 21 des Instruments mit dem optischen Sensor 22 dargestellt. Der optische Sensor bzw. das Ende des Instruments umfasst weiter einen nicht dargestellten optischen Projektor, der z.B. als Laserprojektionsgerät ausgeführt sein kann, um ein Projektionsbild 31 zu projizieren. Ist das Instrument, wie dargestellt, in Richtung des im Gehörgang 11 endständig angeordneten Trommelfells 12 ausgerichtet, so wird auch das Projektionsbild 31 im Bereich des Trommelfells projiziert. Das Projektionsbild kann z.B. eine Fläche oder mehrere Linien umfassen. Im dargestellten Ausführungsbeispiel wird eine Linie projiziert, deren Kontur der Kontur des Trommelfells 12 gleicht. Das Trommelfell 12 erscheint üblicherweise annähernd kreisrund oder oval, so dass also auch ein rundes oder ovales Projektionsbild 31 projiziert wird. Die Projektion des Projektionsbildes 21 ist durch gestrichelte Linien angedeutet. Der Blickwinkel, unter dem vom optischen Sensor 22 aus betrachtet, das Trommelfell 12, ist ebenfalls durch Linien angedeutet.

Aus der optischen Geometrie von Abbildungen ist geläufig, dass der Blickwinkel, unter dem das Trommelfell 12 erscheint, mit abnehmender Distanz D zwischen Sensor 22 und Trommelfell 12 größer wird. Andererseits ist ersichtlich, dass der Projektionswinkel der Projektionsabbildung wegen der Form des Gehörgangs 11 mit abnehmender Distanz kleiner wird. In der gewählten und beispielhaft dargestellten Abbildungsgeometrie erscheint die Projektionsabbildung also zunächst größer als die Abbildung des Trommelfells 12; mit abnehmender Distanz gleichen sich die beiden Größen jedoch an.

Zudem sind der optische Sensor 22 und der Projektor nicht auf einer gemeinsamen optischen Achse angeordnet, so dass der Projektionswinkel zur Ausrichtung des optischen Sensors 22 nicht parallel ist (Parallaxe). Anhand des bekannten zwischenliegenden Winkels und des Abstands des Projektiosabbilds 31 zur Mitte eines vom optischen Sensor 22 aufgenommenen Abbildes kann ein Maß für die absolute Distanz zum Trommelfell 12 ermittelt werden.

In der **Fig. 3** ist zur Illustration dargestellt, wie sich die Größe, unter der das Trommelfell 12 in der Abbildung erscheint, mit abnehmender Distanz verhält. Je nach Eigenschaften der Abbildungsoptik verändert sich die Größe in der Abbildung linear mit der Distanz.

In der **Fig. 4** ist eine der vorangehend beschriebenen Fig. 2 vergleichbare Konstellation dargestellt. Der Tubus oder Schlauch 21 des Instruments ist so auf das Trommelfell 12 ausgerichtet, dass der optische Sensor diesem gegenübersteht. Eine nicht dargestellte Vorrichtung zur optischen Projektion projiziert daher in Richtung auf das Trommelfell 12 ein Projektionsbild 31. Das Projektionsbild 31 ist in seiner Kontur der Kontur des Trommelfells 12 insofern ähnlich, als es im Wesentlichen rund bzw. oval ist. Wie vorangehend erläutert, ändern sich die Größen des Trommelfells 12 und des Projektionsbildes 31 in der Abbildung in unterschiedlicher Weise, wenn die Distanz zwischen dem Ende des Tubus oder Schlauches 21 und dem Trommelfell 12 verändert wird. Die Unterschiede der jeweiligen Kontur lassen sich durch beispielhaft eingezeichnete Abstände d1, d2, d3 erfassen. Diese Abstände oder ein ausgewählter aus diesen Abständen oder ein Mittelwert oder ähnliches, lassen sich daher dazu verwenden, ein Maß für die Distanz zwischen dem optischen Sensor 22 und dem Trommelfell 12 zu ermitteln.

In **Fig. 5** ist schematisch ein Abbild des Trommelfells 12 dargestellt, das durch den optischen Sensor 22 gewonnen wird. Das Trommelfell 12 nimmt in der Abbildung eine bestimmte Fläche A2 ein, die von der Distanz des optischen Sensors 22 zum Trommelfell 12 abhängig ist. Wie vorangehend erläutert, nimmt diese Fläche mit abnehmender Distanz zu. Die verbleibende Fläche A1, die das Trommelfell 12 nicht beinhaltet, nimmt entsprechend mit abnehmender Distanz ab. Daher lässt sich aus dem Verhältnis von A1 zu A2 ein Maß für die Distanz ermitteln; genauer gesagt, nimmt das Verhältnis von a1 zu a2 mit abnehmender Distanz ab.

Wie vorangehend erläutert, kann insbesondere durch Verwendung eines Projektionsbildes, z.B. durch Laserprojektion, aus den Bilddaten des Projektionsbildes und des Trommelfells 12, im Abbild des optischen Sensors ein sehr genaues, eventuell sogar absolutes Maß für die Distanz zwischen Sensor und Trommelfell ermittelt werden. Voraussetzung hierfür sind geeignete optische geometrische Verhältnisse, unter denen das Projektionsbild in einem vorbestimmten Winkel zur zentralen Achse des Instruments, z.B. Otoskops, projiziert wird. Die Verwendung einer solchen optischen Projektion dient also einerseits einer möglichst exakten Messung der Distanz; andererseits ist sie insbesondere bei Instrumenten ohne zusätzlichen Aufwand implementierbar, die den Gehörgang auf Basis entsprechender Projektionsdaten scannen.

Derartige Verfahren sind beispielsweise üblich zur Erfassung von 3D-Abbildern des Gehörgangs, auf deren Basis IdO-Hörhilfen oder Otoplastiken angefertigt werden, die möglichst exakt an die individuelle Gehörgangsform angepasst sein sollen. Damit werden für die Optimierung der Gehäuseform sowie für die akustische Optimierung des Gehörgeräts die gleichen optischen Komponenten verwendet, wie für die Distanzbestimmung. Lediglich die Signalverarbeitung muss zusätzlich zur Ermittlung der Projektionsabbildung in Bezug auf die Distanz-Ermittlung sowie zur Ermittlung der Trommelfell-Kontur angepasst werden.

Ein Grundgedanke der Erfindung lässt sich wie folgt zusammenfassen: Die Erfindung betrifft eine Vorrichtung und ein Verfahren zum Messen der Distanz zum Trommelfell. Die Vorrichtung umfasst einen optischen Sensor 22 und eine Signalverarbeitungseinrichtung 23. Gemäß einem Grundgedanken der Erfindung erfasst der optische Sensor 22 ein Abbild des Trommelfells 12, und die Signalverarbeitungseinrichtung 23 ermittelt in dem Abbild eine quer zu der zu messenden Distanz D erstreckte Fläche A1,A2 oder Abstand d1,d2,d3 und ermittelt in Abhängigkeit von der ermittelten Fläche A1,A2 oder Abstand d1,d2,d3 die Distanz D zu Trommelfell 12. Das Verfahren umfasst gemäß einem Grundgedanken der Erfindung die Schritte S1 Erfassen eines Abbilds des Trommelfells 12, S2 in dem Abbild Ermitteln einer quer zu der zu messenden Distanz D erstreckten Fläche A1,A2 oder Abstand d1,d2,d3, und S3 Ermitteln der Distanz D in Abhängigkeit von der ermittelten Fläche A1,A2 oder Abstand d1,d2,d3. In einer vorteilhaften Weiterbildung wird eine Projektionsabbildung auf oder in die Nähe des Trommelfells 12 projiziert; dann kann die Distanz D vorteilhaft aus dem Abstand d1,d2,d3 zwischen der Projektionsabbildung 31 und dem Trommelfell 12 oder der Bildmitte des Abbilds ermittelt werden. Vorteilhafterweise kann ein eventuell zu anderen Zwecken ohnehin vorhandener optischer Sensor verwendet werden. Vorteilhafterweise stehen Bildverarbeitungsalgorithmen zur Analyse von Bilddaten hinsichtlich gewöhnlicher geometrischer Abbildungsmerkmale, nämlich Fläche oder Abstand, zur Verfügung und erfordern keine besondere Implementierung auf Vorrichtungsseite. Zudem arbeiten solche Bildverarbeitungsalgorithmen weitgehend unabhängig von Störungen der Bildqualität.

## Patentansprüche

1. Vorrichtung zur Messung einer Distanz (D) zwischen dem distalen Ende eines Instruments (20), das zum Einführen in einen Gehörgang ausgebildet ist, und einem endständigen Abschluss, insbesondere dem Trommelfell (12), eines Gehörgangs, in den das Instrument (20) eingeführt ist, umfassend einen optischen Sensor (22) und eine Signalverarbeitungseinrichtung (23),
**dadurch gekennzeichnet, dass**
der optische Sensor (22) ein Abbild des endständigen Abschlusses erfasst, dass die Signalverarbeitungseinrichtung (23) in dem Abbild eine quer zu der zu messenden Distanz (D) erstreckte Fläche (A1,A2) oder Abstand (d1,d2,d3) ermittelt und in Abhängigkeit von der ermittelten Fläche (A1,A2) oder Abstand (d1,d2,d3) die Distanz (D) ermittelt.

2. Vorrichtung nach Anspruch 1,
**dadurch gekennzeichnet, dass**
die optische Apertur des optischen Sensors (22) konstant ist.

3. Vorrichtung nach einem der vorhergehenden Ansprüche,
**dadurch gekennzeichnet, dass**
die Signalverarbeitungseinrichtung (23) die Distanz (D) in zusätzlicher Abhängigkeit von der optischen Apertur des optischen Sensors (22) ermittelt.

4. Vorrichtung nach einem der vorhergehenden Ansprüche,
**dadurch gekennzeichnet, dass**
die Signalverarbeitungseinrichtung (23) in dem Abbild eine Fläche (A2) des endständigen Abschlusses ermittelt und in Abhängigkeit von dieser Fläche (A2) die Distanz (D) ermittelt.

5. Vorrichtung nach einem der vorhergehenden Ansprüche,
**dadurch gekennzeichnet, dass**
die Signalverarbeitungseinrichtung (23) in dem Abbild eine den endständigen Abschluss nicht beinhaltende Fläche (A1) ermittelt und in Abhängigkeit von dieser Fläche (A1) die Distanz (D) ermittelt.

6. Vorrichtung nach einem der vorhergehenden Ansprüche,
**dadurch gekennzeichnet, dass**
sie einen optischen Projektor umfasst, der dazu ausgebildet ist, eine vorbestimmte Projektionsabbildung (31) zu projizieren, dass der optische Projektor so angeordnet ist, dass er die Projektionsabbildung (31) in Richtung des endständigen Abschlusses projiziert, dass die Signalverarbeitungseinrichtung (23) in dem Abbild einen Abstand (d1,d2,d3) zwischen der Projektionsabbildung (31) und dem endständigen Abschluss ermittelt und in Abhängigkeit von diesem Abstand (d1,d2,d3) die Distanz (D) ermittelt.

7. Vorrichtung nach Anspruch 6,
**dadurch gekennzeichnet, dass**
die vorbestimmte Projektionsabbildung (31) in Anpassung an die erwartete Kontur des endständigen Abschlusses konturiert ist.

8. Vorrichtung nach einem der Ansprüche 6 oder 7,
**dadurch gekennzeichnet, dass**
die Signalverarbeitungseinrichtung (23) in dem Abbild einen Abstand (d1,d2,d3) zwischen einer Kontur der Projektionsabildung (31) und einer Kontur des endständigen Abschlusses ermittelt und in Abhängigkeit von diesem Abstand (d1,d2,d3) die Distanz (D) ermittelt.

9. Verfahren zur Messung einer Distanz (D) zwischen dem distalen Ende eines Instruments (20), das zum Einführen in einen Gehörgang ausgebildet ist, und einem endständigen Abschluss, insbesondere dem Trommelfell, eines Gehörgangs, in den das Instrument (20) eingeführt ist,
**umfassend die Schritte**
(S1) Erfassen eines Abbilds des endständigen Abschlusses
(S2) in dem Abbild Ermitteln einer quer zu der zu messenden Distanz (D) erstreckten Fläche (A1,A2) oder Abstand (d1,d2,d3)
(S3) Ermitteln der Distanz (D) in Abhängigkeit von der ermittelten Fläche (A1,A2) oder Abstand (d1,d2,d3).

10. Verfahren nach Anspruch 9,
wobei beim Ermitteln der Distanz (D) zusätzlich eine Abhängigkeit von der optischen Apertur des zum Erfassen des Abbildes verwendeten optischen Sensors (22) berücksichtigt wird.

11. Verfahren nach einem der Ansprüche 9 oder 10,
**umfassend die Schritte**
(S4) in dem Abbild Ermitteln einer Fläche (A2) des endständigen Abschlusses
(S5) Ermitteln der Distanz (D) in Abhängigkeit von dieser Fläche (A2).

12. Verfahren nach einem der Ansprüche 9 bis 11,
**umfassend die Schritte**
(S6) in dem Abbild Ermitteln einer den endständigen Abschluss nicht beinhaltenden Fläche (A1)
(S7) Ermitteln der Distanz (D) in Abhängigkeit von dieser Fläche (A1).

13. Verfahren nach nach einem der Ansprüche 9 bis 12,
**umfassend die Schritte**
(S8) Projizieren einer vorbestimmten Projektionsabbildung (31) in Richtung des endständigen Abschlusses
(S9) in dem Abbild Ermitteln eines Abstands (d1,d2,d3) zwischen der Projektionsabbildung (31) und dem endständigen Abschluss
(S10) Ermitteln der Distanz (D) in Abhängigkeit von diesem Abstand (d1,d2,d3).

14. Verfahren nach Anspruch 13,
wobei die vorbestimmte Projektionsabbildung (31) in Anpassung an die erwartete Kontur des endständigen Abschlusses konturiert ist.

15. Verfahren nach einem der Ansprüche 13 oder 14,
**umfassend die Schritte**
(S11) in dem Abbild Ermitteln eines Abstands (d1,d2,d3) zwischen einer Kontur der Projektionsabbildung (31) und einer Kontur des endständigen Abschlusses
(S12) Ermittelt der Distanz (D) in Abhängigkeit von diesem Abstand (d1,d2,d3).
